# EUROPEAN PATENT SPECIFICATION

(11) **EP 0 956 860 B1**
(45) Date of publication and mention of the grant of the patent: **18.02.2004**
(21) Application number: 97944185.4
(22) Date of filing: 20.10.1997
(51) Int. Cl.: A61K 35/78, A23L 1/30, A23L 2/38, A61P 37/02

(54) **HEMATOPOIETIC FUNCTION RESTORATIVE AGENT**
Mittel zur Wiederherstellung der hämatopoetischen Funktion
PRODUIT RESTITUANT LA FONCTION HEMATOPOIETIQUE

(30) Priority: 21.10.1996 JP 27830996
(43) Date of publication of application: 17.11.1999
(73) Proprietor: Yahagi, Manami, Yamagata 999-5604 (JP); Yoshihara, Akio, Saitama 353-0007 (JP)
(72) Inventor: YOSHIHARA, Akio, Shiki-shi, Saitama 353-0007 (JP); YAHAGI, Nobuo, Mogami-gun, Yamagata 999-5604 (JP); FUSHIYA, Shinji, Sendai-shi, Miyagi 980-0022 (JP); TAKANO, Fumihide, Sendai-shi, Miyagi 982-0011 (JP); HOJO, Hiroshi, Kawasaki-shi, Kanagawa 214-0014 (JP)
(74) Representative: Harding, Charles Thomas
(86) International application number: PCT/JP1997/003787
(87) International publication number: WO 1998/017296

(56) References cited:
- EP-A- 0 491 114
- WO-A-94/16715
- WO-A-95/04481
- BE-A- 617 664
- GB-A- 776 722
- JP-A- 1 165 356
- JP-A- 3 273 098
- JP-A- 7 289 197
- JP-A- 48 010 273
- JP-A- 54 005 053
- JP-A- 57 206 391
- DATABASE WPI Section Ch, Week 199713 Derwent Publications Ltd., London, GB; Class B04, AN 1997-133378 XP002196106 & CN 1 090 143 A (XIANGFAN FOOD FACTORY HUBEI PROV), 3 August 1994 (1994-08-03)
- DATABASE WPI Section Ch, Week 199014 Derwent Publications Ltd., London, GB; Class A96, AN 1990-104534 XP002196107 & JP 02 056435 A (GREEN CROSS CORP), 28 February 1990 (1990-02-28)
- SONI S K ET AL: "Extracellular amylase production by Saccharomycopsis capsularis and its evaluation for starch saccharification." FOLIA MICROBIOLOGICA, vol. 41, no. 3, 1996, pages 243-248, XP001068856 ISSN: 0015-5632
- EZEOGU LEWIS I ET AL: "Effect of molasses concentration and medium supplementation on the adaptability and viability of a high level ethanol-tolerant palm-wine Saccharomyces isolate." BIOTECHNOLOGY LETTERS, vol. 16, no. 1, 1994, pages 95-100, XP001068811 ISSN: 0141-5492
- SUZUKI I ET AL: "ANTITUMOR AND IMMUNOMODULATING ACTIVITIES OF A BETA GLUCAN OBTAINED FROM LIQUID-CULTURED GRIFOLA-FRONDOSA" CHEMICAL & PHARMACEUTICAL BULLETIN (TOKYO), vol. 37, no. 2, 1989, pages 410-413, XP001068921 ISSN: 0009-2363
- WANG H X ET AL: "Immunomodulatory and antitumor activities of a polysaccharide-peptide complex from a mycelial culture of Tricholoma sp., a local edible mushroom." LIFE SCIENCES, vol. 57, no. 3, 1995, pages 269-281, XP001069204 ISSN: 0024-3205
- XU R ET AL: "EFFECT OF CORDYCEPS-SINENSIS ON MURINE HEMATOPOIETIC STEM CELLS" HUNAN YIKE DAXUE XUEBAO, vol. 15, no. 1, 1990, pages 48-50, XP001068810 ISSN: 1000-5625
- YANG MABEL MEI PO ET AL: "The anti-tumor effect of a small polypeptide from Coriolus versicolor (SPCV)." AMERICAN JOURNAL OF CHINESE MEDICINE, vol. 20, no. 3-4, 1992, pages 221-232, XP001068845 ISSN: 0192-415X
- DATABASE WPI Section Ch, Week 199343 Derwent Publications Ltd., London, GB; Class C05, AN 1993-339605 XP002196108 & JP 05 246816 A (SOLAR JAPAN KK), 24 September 1993 (1993-09-24)
- ZHANG X, LING L, DAI R: "Constituents of the seed coat of Arachis hypogaea L." ZHONGGUO ZHONG YAO ZA ZHI = CHINA JOURNAL OF CHINESE MATERIA MEDICA, vol. 15, no. 6, June 1990 (1990-06), pages 356-358, 384, XP001069304

## Description

### TECHNICAL FIELD

The present invention relates to an agent for recovering hematopoietic function and a processed food both containing a treated product of peanut seed coats.

### BACKGROUND ART

Antibiotic-type carcinostatics used in chemotherapy for cancer damage the function of bone marrow when administered for a long time, causing hematopoietic dysfunctions (represented by thrombocytopenia) and leukopenia. Among these side effects, the inhibition of lymphocyte functions associated with leukopenia causes remarkable decrease in natural curing ability in humans and infectious diseases in them. Thus, this side effect is a big obstacle to recovery from diseases.

In order to alleviate those side effects occurring in chemotherapy for cancer, recombinant formulations of cytokine genes which activate lymphocyte functions, such as human granulocyte colony-stimulating factor (rhG-CSF), human granulocyte-macroph age colony-stimulating factor (rhGM-CSF) and human macrophage colony-stimulating factor (rhM-CSF), are applied. However, since these medicines are protein formulations prepared by recombinant DNA technology, their cost per day is rather high and yet there are limitations in the term of use and in the administration method (intravenous injection). Therefore, development of effective medicines which are cheap and capable of oral administration is desired.

### DISCLOSURE OF THE INVENTION

It is an object of the present invention to provide an agent for recovering hematopoietic function and a processed food both comprising a physiologically active substance which is derived from a natural product and capable of oral administration.

As a result of extensive search for a physiologically active substance having bone marrow cell-proliferating activity which occurs in the natural world and does not cause side effects, the present inventors have found that a water extract from peanut seed coats has an effect of proliferating bone marrow cells. Further, it was found that the extract exhibits the above-mentioned effect even when administered orally and, thus, it is useful as an agent for recovering hematopoietic function and as a processed food. Thus, the present invention has been achieved.

The present invention includes the following inventions.

Use of a water extract of peanut seed coats for the preparation of a pharmaceutical agent for recovering hematopoietic function.

Use of a water extract of peanut seed coats for the preparation of a processed food for recovering hematopoietic function.

Preferably, the water extract according to the present invention is a cold water immersion extract.

Suitably, the cold water immersion extract according to the present invention is a water extract obtained at room temperature.

Preferably, the agent for recovering hematopoietic function according to the present invention is used as a processed food.

The fruit of peanut has a solid pericarp. Usually, there are two seeds present inside the pericarp. These seeds are covered with seed coats. In the present invention, these seed coats are used.

In the present invention, a water extract of peanut seed coats is used.

The solvent of the present invention is water.

In the extraction, peanut seed coats may be used without any treatment. Alternatively, they may be crushed or powdered to give greater contact with the solvent.

A waste water generated in the process of separating peanut seed coats from peanut seeds by washing them with water and then purified or dried if necessary may be used as an extract. However, it is preferable to extract peanut seed coats with the solvent in an amount of 5 to 25 liters per kg of the coats.

Extraction temperature ranges preferably from room temperature to the boiling point of the solvent under ambient pressure. Extraction period is preferably one day though it varies depending on the extraction temperature.

The thus obtained extract as it is may be used as. a component of the agent or of the processed food for recovering hematopoietic function. Alternatively, the extract may be treated by various means of purification such as ion exchange chromatography, gel filtration chromatography, dialysis, etc. to obtain active fractions using bone marrow cell-proliferating activity as an indicator. Then, such a treated extract with enhanced activity may be used in the invention.

The agent for recovering hematopoietic function can be formulated into pharmaceutical preparations by using a water extract from peanut seed coats alone or in combination with a pharmaceutical carrier. The dosage form is not particularly limited and may be appropriately selected according to the route and method of administration. Generally, the agent is used as oral agents such as tablets, capsules, granules, fine granules or powder.

The dosage of the agent for recovering hematopoietic function varies depending on the age, weight and degree of the disease of a patient. In oral administration, the dosage ranges usually from 100 to 500 mg per day as dry powder of peanut seed coat extract. Usually, the number of administration is once to three times a day in oral administration.

Such oral agents and processed foods are prepared by conventional methods using excipients starch, lactose, sucrose, mannitol, carboxymethylcellulose, corn starch, inorganic salts, etc.

In such oral agents and processed foods, binders, disintegrants, surfactants, lubricants, fluidity promoters, flavoring agents, coloring agents, perfumes and the like may also be used appropriately in addition to the above-mentioned excipient.

As binders, crystalline cellulose, crystalline cellulose/carmellose sodium, methylcellulose, hydroxypropylcellulose, hydroxypropylcellulose with a low degree of substitution, hydroxypropylmethylcellulose 2208, hydroxypropylmethylcellulose 2906, hydroxypropylmethylcellulose 2910, hydroxypropylmethylcellulose phthalate 200731, hydroxypropylmethylcellulose phthalate 220824, hydroxypropylmethylcellulose acetate succinate, carmellose sodium, ethylcellulose, carboxymethylethylcellulose, hydroxyethylcellulose, wheat starch, rice starch, corn starch, potato starch, dextrin, gelatinized starch, partially gelatinized starch, hydroxypropylstarch, pullulan, polyvinylpyrrolidone K25, polyvinylpyrrolidone K30, aminoalkyl methacrylate copolymer E, aminoalkyl methacrylate copolymer RS, methacrylate copolymer L, methacrylate copolymer S, methacrylate copolymer LD, polyvinyl acetal diethyl aminoacetate, polyvinyl alcohol, acacia, powdered acacia, agar, gelatin, white shellac, tragacanth gum, purified sucrose, macrogol 200, macrogol 300 and macrogol 6000 may be enumerated, for example.

As disintegrants, crystalline cellulose, methylcellulose, hydroxypropylcellulose with a low degree of substitution, carmellose, carmellose calcium, carmellose sodium, cross-carmellose sodium, wheat starch, rice starch, corn starch, potato starch, partially gelatinized starch, hydroxypropyl starch, sodium carboxymethyl starch and tragacanth gum may be enumerated, for example.

As surfactants, soybean lecithin, sucrose fatty acid esters, polyoxyl stearate 40, polyoxyethylene hardened castor oil 100, polyoxyethylene hardened castor oil 40, polyoxyethylene hardened castor oil 50, polyoxyethylene hardened castor oil 60, polyoxyethylene[42] polyoxypropylene[67] glycol, polyoxyethylene[54] polyoxypropylene[39] glycol, polyoxyethylene[105] polyoxypropylene[5] glycol, polyoxyethylene[160] polyoxypropylene[80] glycol, polyoxyethylene[196] polyoxypropylene[67] glycol, sorbitan sesquioleate, sorbitan trioleate, sorbitan monostearate, sorbitan monopalmitate, sorbitan monolaurate, polysorbate 40, polysorbate 60, polysorbate 65, polysorbate 80, glycerol monostearate, sodium lauryl sulfate and lauromacrogol may be enumerated, for example.

As lubricants, wheat starch, rice starch, corn starch, stearic acid, calcium stearate, magnesium stearate, hydrous silicon dioxide, light anhydrous silicic acid, synthetic aluminium silicate, dry aluminium hydroxide gel, talc, magnesium alminate metasilicate, calcium hydrogenphosphate, anhydrous calcium hydrogenphosphate, sucrose fatty acid esters, waxes, hydrogenated vegetable oils and polyethylene glycol may be enumerated, for example.

As fluidity promoters, hydrous silicon dioxide, light anhydrous silicic acid, dry aluminium hydroxide gel, synthetic aluminium silicate and magnesium silicate may be enumerated, for example.

The agent and the processed food for recovering hematopoietic function may also be administered as a suspension, emulsion, syrup or elixir. These formulations may contain flavoring agents, aromatics and coloring agents.

Peanut seed coats which is a raw material for preparing the agent and the processed food for recovering hematopoietic function are served, together with peanut seeds, as food. Thus, the safety of this material has been established.

### BRIEF DESCRIPTION OF THE DRAWINGS

Fig. 1 shows the effect of extract from peanut seed coats on bone marrow cell-proliferating activity.
Fig. 2 shows the concentration dependency of the bone marrow cell-proliferating activity of extract from peanut seed coats.
   AHPE: cold immersion extract from peanut seed coats
   AHPHE: hot water extract from peanut seed coats
   AH seed extract: cold immersion extract from peanut seeds
   Saline: cell proliferation when cells are cultured in saline alone

### BEST MODES FOR CARRYING OUT THE INVENTION

The present invention will be described more specifically below with reference to the following Examples.

### EXAMPLE 1.

### (1) Preparation of Extract from Peanut Seed Coats

Seeds of peanut (*Arachis hypogaea)* (Leguminosae) produced in China were dried for about one month, and then their seed coats were separated. Twenty-five grams of the dried seed coat was weighed accurately and placed in a 2 liter Erlenmeyer flask. One liter of purified water was added thereto and allowed standing at room temperature (22± 3 °C ) for 24 hours. The resultant mixture was filtered with a filter cotton, and the filtrate was freeze-dried to thereby obtain peanut seed coat extract powder (yield: 4.4484 g per 25 g of peanut seed coats). This powder is hygroscopic, reddish-brown, fine powder without smell and with a bitter taste. This powder is readily soluble in both water and ethanol.

### (2) Experiment on Bone Marrow Cell Proliferating Activity

Mouse bone marrow cells were used in this experiment. Briefly, C57BL/6 mice (specific pathogen free, female, 5-6 week old; purchased from SLC Japan) were slaughtered under excessive etherization. Two thigh bones were removed from each mouse aseptically. Subsequently, a 2.5 ml syringe (Terumo Corp.) provided with a 23 gauge needle (Terumo Corp.) was filled with α-MEM (commercial product of Gibco; containing 20% fetal bovine serum, 5% bovine serum albumin, 50 µM 2-mercaptoethanol) medium for culturing bone marrow cells. The upper edge of the thigh bone was pierced with the above-mentioned needle to thereby separate bone marrow cells. The resultant bone marrow cells were arranged to give a concentration of 5 x 10⁴ cells/ml and added to 96-well culture plates (Becton Dickinson). To this cell culture fluid, the extract from peanut seed coats (obtained in (1) above; hereinafter referred to as the "cold immersion extract") which was diluted with saline to give a concentration of 25-200 µg/ml or saline alone as a control was added. Then, cells were cultured for 14 days.

Bone marrow cell-proliferation was judged by colorimetry using MTT (3-(4,5-dimethylthiazole-2-yl)-2,5-diphenyltetrazolium bromide) which is a common method for assaying cell proliferation ratios.

As positive controls for bone marrow cell proliferation effect, interleukin 3 (IL-3; WEHI3 mouse lymphoma culture supernatant) and interleukin 6 (IL-6; mouse recombinant formulation from Genzyme) of which proliferation effect on bone marrow cells had been confirmed were used.

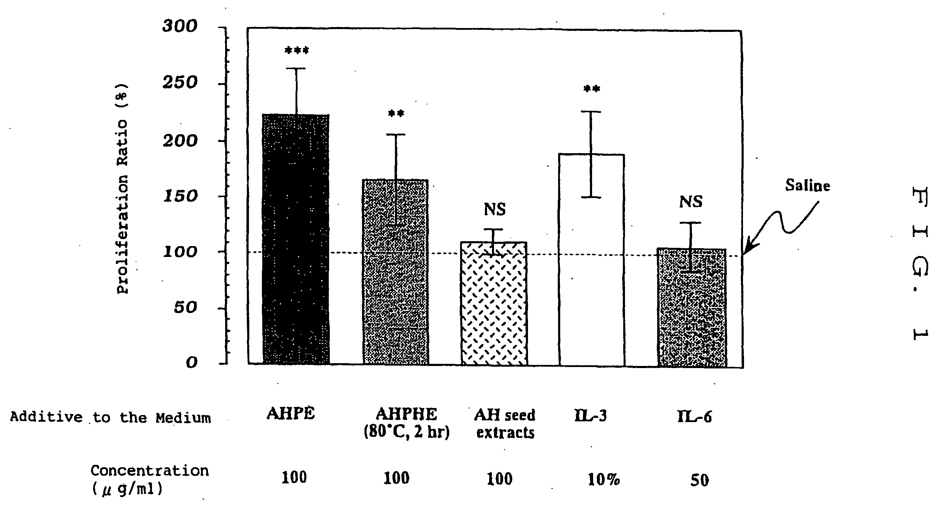

The results are shown in Fig. 1. In these data, the cell proliferation ratio when cultured by saline alone (see Saline in the Figure) was taken as 100%. Proliferation ratios (%) against this value were expressed in mean value ± standard deviation value. Marks "**" and "***" represent statistically (Student t test) significant difference against the result obtained by culturing with saline alone. These marks were given to those experimental results of significance levels less than 1% and less than 0.1%, respectively. Mark "NS" was given when the results exceeded the above-mentioned significance levels and no statistically significant difference was recognized.

Addition of the cold immersion extract from peanut seed coats exhibited bone marrow cell-proliferating activity 2 times greater (see the column "AHPE" in Fig. 1) than that obtained when saline alone (control) was added (proliferation ratio: 100%). This activity of the extract was found to be greater than the activity of IL-3 and IL-6, the cytokines used as positive controls (Fig. 1).

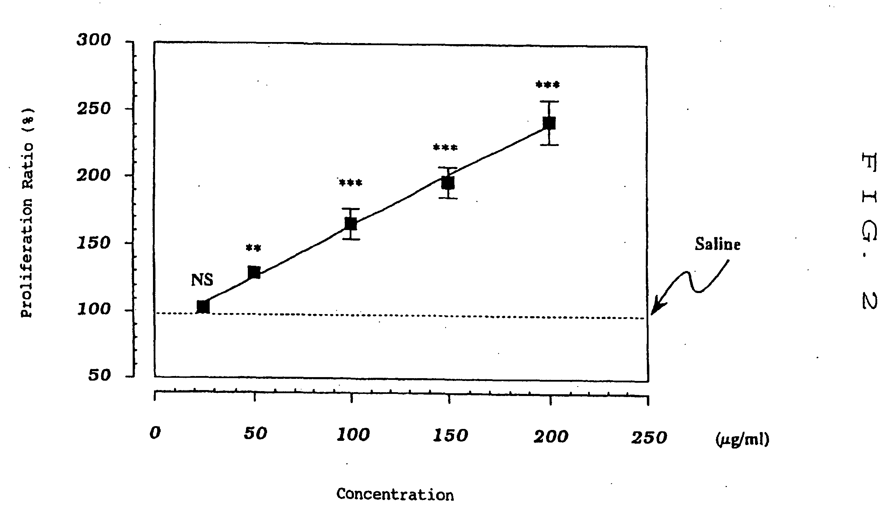

The results of an experiment in which the concentration of the cold immersion extract was varied from 25 to 200 µg/ml are shown in Fig. 2. Concentration dependency was recognized in the activity of the cold immersion extract from peanut seed coats. At a concentration of 70 µg/ml or above, remarkable bone marrow cell-proliferating activity was recognized. Marks in Fig. 2 are the same as defined in Fig. 1.

It should be noted that no bone marrow cell-proliferating activity was recognized in extract from peanut seeds from which seed coats were removed (Fig. 1). Thus, it has become clear that this proliferating activity is the activity of peanut seed coats. Bone marrow cell-proliferating activity was also examined on extract powder which was obtained by extracting peanut seed coats with hot water of 80 °C for 2 hours to improve the extraction efficiency. As a result, it was found that the hot water extract had lower bone marrow cell-proliferating activity than the cold immersion extract. Therefore, it is considered that the cold immersion extract is more effective as an agent for recovering hematopoietic function.

### (3) Human Clinical Data when the Cold Immersion Extract was Used

The number of platelets in the blood in a normal human is 200,000 to 350,000.
Case A: The cold immersion extract powder obtained in (1) above was administered orally at a dosage of 500 mg/day to a 40-year old male whose number of platelets decreased to 30,000 because of the side effects of a carcinostatic and whose life was endangered. As a result, the platelet number was recovered to 350,000 on day 4 of the administration.
Case B: The extract powder was administered at a dosage of 150 mg/day to a 12-year old boy with leukemia for 4 days. As a result, the number of platelets become normal and his fever went down.
Case C: The extract powder was administered orally at a dosage of 150 mg/day for 15 days to a 60-year old female whose number of platelets decreased to 70,000 because of the side effects of a carcinostatic. As a result, the platelet number was recovered to 200,000.
Case D: A 69-year old male having primary pancreatic cancer which had metastasized to the liver was diagnosed to have disseminated intravascular coagulation (DIC) syndrome on January 27. The number of platelets of this patient was 10,000. From the afternoon of January 29, administration of the cold immersion extract powder obtained in (1) above was started. Briefly, 500 mg of the extract powder was dissolved. One nineth (1/9) of this solution was administered orally 3 times a day. On January 30, the platelet number became 7,000; on February 1, the platelet number became 9,000; on February 3, the platelet number became 50,000; and on February 7, this number was recovered to 60,000.

In addition to the above cases, there are a number of clinically effective human cases. The extract powder was also effective for treating hematopoietic dysfunctions such as thrombocytopenia in purpura.

### EXAMPLE 2.

Peanut seed coats (1100 mg) were immersed in 100 ml of hot water (85 °C) for 1 min and 30 sec. Then, the resultant liquid with bloody red color was filtered, and the filtrate was filled in a bottle. The bottle was heated at 110°C for 10 min to thereby degass the liquid in it. After the degassing, a stainless cap was put on the bottle, which was stoppered tightly. The liquid in the tightly stoppered bottle was dipped in hot water of 90 °C for 2 hours for sterilization. As a result of the heating operation, the liquid in the bottle became a clear drink presenting bloody red color.

### EXAMPLE 3.

Peanut seed coats (1500 mg) was decocted in 500 ml of water in an earthen teapot over a low fire so that a half of the water was evaporated in 30 min. The resultant bloody red liquid was filtered to obtain the filtrate as a dosage per day per human.

### EXAMPLE 4.

Peanut seed coats were immersed in hot water (85 °C ) for 1 min and 30 sec. Then, the seed coats were removed from the liquid and washed with water until no bloody red color from the extract came out. The thus washed seed coats were dried completely with hot air of 80°C. To 200 g of the dried seed coats, 10 liters of water and 5 g of sodium hydrogencarbonate were added and reacted at 90°C for 60 min under agitation. The filtrate was heated under refluxing for 60 min and then freeze-dried to thereby obtain powder which was about 0.371% of the dried seed coats.

The thus obtained powder can be used as it is as an active ingredient of the agent for recovering hematopoietic function, or it can be used as a raw material for preparing the processed food for recovering hematopoietic function.

### EXAMPLE 5 - for information purposes only

A sterilized medium was prepared by adding to peanut seed coats 0.1% vitamins and 0.1% minerals. To this medium, spores of a basidiomycete (e.g. polypore) or ascomycete (e.g. *Cordyceps* fungus) were inoculated and cultured. After the maturation of hyphae, fruit bodies were formed. The fruit bodies and aggregations of the fungus were dried and powdered.

The thus obtained powder can be used as it is as an active ingredient of the agent for recovering hematopoietic function, or it can be used as a raw material for preparing the processed food for recovering hematopoietic function.

### EXAMPLE 6 - for information purposes only

A sterilized medium was prepared by adding to peanut seed coats vitamins, minerals and saccharides. To this medium, spores of a basidiomycete (e.g. polypore) or ascomycete (e.g. *Cordyceps* fungus) were inoculated and cultured. After the maturation of hyphae, fruit bodies were formed. The fruit bodies and the entire medium were boiled in hot water of 90 °C for 120 min under agitation. The filtrate was heated under refluxing for another 60 min, and then freeze-dried to obtain powder.

The thus obtained powder can be used as it is as an active ingredient of the agent for recovering hematopoietic function, or it can be used as a raw material for preparing the processed food for recovering hematopoietic function.

### EXAMPLE 7 - for information purposes only

The freeze-dried powder obtained in Example 6 (400 mg) was filled in a 350 ml bottle and degassed. The bottle was stoppered tightly and finally sterilized to prepare a drink.

### EXAMPLE 8.

Peanut seed coats were placed in 99.9% ethanol at 18 °C in such a manner that the precipitated seed coats occupied 30% of the volume of the ethanol, and left stationary for 40 days. Then, the extract was freeze-dried to prepare powder.

The thus obtained powder can be used as it is as an active ingredient of the agent for recovering hematopoietic function, or it can be used as a raw material for preparing the processed food for recovering hematopoietic function.

### EXAMPLE 9.

The powder obtained in Example 8 (10 g) was mixed with 490 g of corn starch and kneaded with addition of water. The resultant material was granulated through a screen of 1 mm x 1 mm meshes and dried to prepare granules.

One gram of the thus obtained granules contains 20 mg of the powder obtained in Example 8. Five to 10 grams of these granules is administered 3 times a day according to the symptoms of a patient.

### INDUSTRIAL APPLICABILITY

The agent for recovering hematopoietic function has an effect of recovering hematopoietic function such as increasing platelet-producing ability, is capable of oral administration and is prepared from a safe food as a raw material. Thus, the field of clinical application of hematopoietic function recovering agent can be enlarged. The agent is especially effective when bone marrow function was decreased due to the use of carcinostatics or radiotherapies or when disorders in platelet production are caused by hepatic diseases or blood diseases.

Further, the processed food also has an effect of recovering hematopoietic function such as increasing platelet-producing ability and is prepared from a safe food as a raw material.

## Claims

1. Use of a water extract of peanut seed coats for the preparation of a pharmaceutical agent for recovering hematopoietic function.

2. The use of claim 1, wherein the water extract is a cold water immersion extract.

3. The use of claim 2, wherein the cold water immersion extract is a water extract obtained at room temperature.

4. The use of any one of the preceding claims, wherein the agent for recovering hematopoietic function is used as a processed food.

5. Use of a water extract of peanut seed coats for the preparation of a processed food for recovering hematopoietic function.

## Patentansprüche

1. Verwendung eines Wasserextrakts von Erdnußkernhüllen für die Herstellung eines pharmazeutischen Mittels zur Gewinnung von Hämatopoesefunktion.

2. Verwendung nach Anspruch 1, bei der der Wasserextrakt ein Kaltwassereintauchextrakt ist.

3. Verwendung nach Anspruch 2, bei der der Kaltwassereintauchextrakt ein bei Raumtemperatur erhaltener Extrakt ist.

4. Verwendung nach einem der vorausgehenden Ansprüche, bei der das Mittel zur Gewinnung von Hämatopoesefunktion als ein verarbeitetes Nahrungsmittel verwendet wird.

5. Verwendung eines Wasserextraktes von Erdnußkernhüllen für die Herstellung eines verarbeiteten Nahrungsmittels zur Gewinnung von Hämatopoesefunktion.

## Revendications

1. Utilisation d'un extrait aqueux d'enveloppes de graines d'arachide pour la préparation d'un agent pharmaceutique destiné au rétablissement de la fonction hématopoïétique.

2. Utilisation suivant la revendication 1, dans laquelle l'extrait aqueux est un extrait obtenu par immersion dans l'eau froide.

3. Utilisation suivant la revendication 2, dans laquelle l'extrait obtenu par immersion dans l'eau froide est un extrait aqueux obtenu à température ambiante.

4. utilisation suivant l'une quelconque des revendications précédentes, dans laquelle l'agent destiné au rétablissement de la fonction hématopoïétique est utilisé sous forme d'un aliment préparé.

5. Utilisation d'un extrait aqueux d'enveloppes de graines d'arachide pour la préparation d'un aliment préparé destiné au rétablissement de la fonction hématopoïétique.
